# EUROPEAN PATENT SPECIFICATION

(11) **EP 3 435 976 B1**
(45) Date of publication and mention of the grant of the patent: **10.03.2021**
(21) Application number: 17740793.9
(22) Date of filing: 03.07.2017
(51) Int. Cl.: A61K 9/00, A61K 47/02, A61K 47/18, A61K 9/08, A61K 31/4375, A61K 31/444, A61P 31/04, A61K 31/4545

(54) **ANTIBACTERIAL COMPOSITIONS**
ANTIBAKTERIELLE ZUSAMMENSETZUNGEN
COMPOSITIONS ANTIBACTÉRIENNES

(30) Priority: 14.04.2017 IN 201721013400
(43) Date of publication of application: 06.02.2019
(73) Proprietor: Wockhardt Limited, M.S. Aurangabad 431006 (IN)
(72) Inventor: CHAUHAN, Bhaskar, Sambhal 202412 Uttar Pradesh (IN); SHAIKH, Mohammed Asif, Aurangabad 431001 Maharashtra (IN); JOSHI, Rajendra Arun, Aurangabad 431107 Maharashtra (IN); HANDA, Ajay Kumar, AHMEDABAD-380015 GUJARAT (IN); YEOLE, Ravindra Dattatraya, Aurangabad 431003 Maharashtra (IN); PATEL, Mahesh Vithalbhai, Aurangabad 431003 Maharashtra (IN)
(74) Representative: Nederlandsch Octrooibureau
(86) International application number: PCT/IB2017/053998
(87) International publication number: WO 2018/189575

(56) References cited:
- WO-A2-00/68229
- WO-A2-2004/058303
- WO-A2-2009/027762

## Description

The invention as defined in claim relates to pharmaceutical compositions and their use in treatment, control or prevention of bacterial infections.

### BACKGROUND OF THE INVENTION

The infections caused by bacteria continue to be an area of significant concern globally. The emergence of bacterial resistance to known antibacterial agents is becoming a major challenge in treating bacterial infections. One way forward to treat bacterial infections, and especially those caused by resistant bacteria, is to develop new antibacterial agents that can overcome the bacterial resistance. Coates et al. (Br. J. Pharmacol. 2007; 152(8), 1147-1154) have reviewed approaches to developing new antibiotics. However, the development of new antibacterial agents is a challenging task. For example, Gwynn et al. (Annals of the New York Academy of Sciences, 2010, 1213: 5-19) have reviewed the challenges in the discovery of antibacterial agents. Several antibacterial agents and/or compositions have been described in the prior art. However, there remains a need for potent antibacterial agents and/or compositions for use in treatment, control and/or prevention of bacterial infections.

Nadifloxacin is an example of a benzoquinolizine carboxylic acid. Nadifloxacin is racemic [(±)-9-fluoro-8-(4-hydroxypiperidin-1-yl)-5-methyl-6,7-dihydro-1-oxo-1H-5H-benzo [i,j]quinolizine-2-carboxylic acid and is disclosed in JP Patent No. 58,90,511 and US Patent No. 4,399,134. Nadifloxacin has an asymmetric carbon atom at the 5-position thereof. RS-(±)-Nadifloxacin comprises two optically active isomers. In describing an optically active compound, the prefixes R and S or D and L are used to denote the absolute configuration of the molecule about its chiral centre(s). The prefixes (+) and (-) or d and 1 are employed to designate the sign of rotation of plane-polarized light by the compound, with (-) or 1 meaning that the compound is levorotatory. A compound prefixed with (+) or d is dextrorotatory. Compounds having a single chiral centre exist as a pair of enantiomers, which are identical except that they are non-superimposable mirror images of one another. A one-to-one mixture of enantiomers is often referred to as a racemic mixture. Racemic RS-(±)-Nadifloxacin derives its biological activity primarily from the S-(-)-enantiomer. The optically active S-(-)-Nadifloxacin [α] D = -312.0 is obtained as disclosed in Chem. Pharm. Bull 44 (1996), page nos. 642-5 and Jpn. Kokai Tokyo Koho JP 63,192,753. The optically active R-(+)-Nadifloxacin, [af_{O} = +312.0, is obtained as disclosed in Jpn.Kokai Tokyo Koho JP 63,192,753. Pharmaceutical compositions of RS-(±)-Nadifloxacin are disclosed in US Patent No. 4,399,134 and US Patent No. 4,552,879. Although these cited patents disclose compositions of RS-(±)-Nadifloxacin for oral, parenteral and topical use, the only commercial product containing RS-(±)-Nadifloxacin as an active antibacterial compound is the commercial product named Acuatim®. Acuatim® is available as a cream and a lotion and incorporates racemic RS-(±)-Nadifloxacin as 1% of its composition for the topical treatment of acne. Acuatim® has several drawbacks. It is intended only for topical use and is registered only for the treatment of acne caused by Propionibacteήum species. S-(-)-nadi floxacin is the main functional isomer, and its antibacterial activity is 64 to 256 times that of the R isomer and twice as much as that of the racemate.

U.S. Patent No 4,399,134 discloses processes for the preparation of nadifloxacin or salts thereof and antibacterially effective pharmaceutical compositions of nadifloxacin. Typical dosage forms include tablets, pills, powders, liquid preparations, suspensions, emulsions, granules, capsules, suppositories, and injectable preparations (solutions, suspensions, etc). U.S. Patent No 6,884,768 discloses solid oral pharmaceutical compositions that includes nadifloxacin, an absorbefacient and taurine compounds. PCT Publication WO 00/68229 describes the S-(-)-optically pure benzoquinolizine carboxylic acid and their pharmaceutically acceptable salts, derivatives, pseudopolymorphs, and hydrates, substantially free of their R-(+)-isomers, processes for their preparation and pharmaceutical compositions.

U.S. Patent Application 20040176337 discloses topical compositions of benzoquinolizine-2-carboxylic acid antimicrobial drug. U.S. Patent Application 20040176321 discloses injectable pharmaceutical composition for intravenous delivery of an active agent that includes RS-(±)-nadifloxacin; S-(-)-nadifloxacin and hydrates thereof; or S-(-)-nadifloxacin arginine and salts thereof. PCT Publication WO 04/00360 describes pharmaceutical compositions of several active ingredients including nadifloxacin for topical use for treatment of dermatosis. PCT Publication WO 2004/058303 A2 describes pharmaceutical composition in aqueous solution form useful for parenteral application having as an active agent S-(-)-9-fluoro-6,7-dihydro-8-(4-hydroxypiperidin-1-yl)-5-methyl-1-oxo-1H,5H-benzo[i,j]quinolizine-2-carboxylic acid, S-(-)-9-fluoro-6,7-dihydro-8-(4-hydroxypiperidin-1-yl)-5-methyl-1-oxo-1H,5H-benzo[ij]quinolizine-2-carboxylic acid 0.2 hydrate or S-(-)-9-fluoro-6,7-dihydro-8-(4-hydroxypiperidin-1-yl)-5-methyl-1-oxo-1H,5H-benzo[i,j]quinolizine-2-carboxylic acid arginine salt-or a benzoquinolizine-2-carboxylic acid antibiotic drug and the processes for the preparation of composition, the use of composition in preparation of a medicament.

US Patent No. 7,164,023 discloses a crystalline S-(-)-9-fluoro-6,7-dihydro-8-(4-hydroxypiperidin-1-yl)-5-methyl-1-oxo-1H,-5H-benzo[i,j]quinolizine-2-carboxylic acid L-arginine salt tetrahydrate and its preparation. S-(-)-nadifloxacin L-arginine salt can be prepared according to literature methods (J. Med. Chem., 2005, 48, 5232- 5242). PCT Publication WO 2013/046061 A1 describes pure S-(-)-9-fluoro-6,7-dihydro-8-(4-hydroxypiperidin-1-yl)-5-methyl-1-oxo-1H,-5H-benzo[i,j]quinolizine-2-carboxylic acid

L-arginine salt tetrahydrate and a process for its preparation. PCT Publication WO 2009/027762 A2 describes the stable solutions suitable for ophthalmic, otic, or nasal administration that include nadifloxacin and pharmaceutically acceptable salts thereof and processes for the preparation of the stable solutions. PCT Publication WO 2009/027762 A2 describes the pharmaceutical composition, comprising S - (-) - nadifloxacin-L-arginine salt and metal chelating agent and the method of preparing the composition and the use of the composition in the manufacture of antibiotic.

### SUMMARY OF THE INVENTION

Accordingly, there are provided pharmaceutical compositions and their use in treatment, control or prevention of bacterial infection in a subject.

In one general aspect, there is provided a pharmaceutical composition in the form of a solution, said solution comprising S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt, said solution having pH within the range of 9 to 10.5.

In another general aspect, there is provided for use of a composition according to the invention in a method for treating, controlling or preventing bacterial infection.

In another general aspect, there is provided a method for treatment, control or prevention of a bacterial infection in a subject, said method comprising administering to said subject a composition according to the invention.

The details of one or more embodiments of the invention are set forth in the description below. Other features, objects and advantages of the invention will be apparent from the following description including claims.

### DETAILED DESCRIPTION OF THE INVENTION

It must be noted that, as used in this specification and the appended claims, the singular forms "a", "an", and "the" include plural referents unless the content clearly dictates otherwise. All references including patents, patent applications, and literature cited in the specification are expressly incorporated herein by reference in their entirety.

The term "pharmaceutically acceptable salt" as used herein refers to one or more salts of a given compound which possesses the desired pharmacological activity of the free compound and which are neither biologically nor otherwise undesirable. In general, the "pharmaceutically acceptable salts" refer to and include those salts that are suitable for use in contact with the tissues of human and animals without undue toxicity, irritation, allergic response and the like, and are commensurate with a reasonable benefit/risk ratio. Pharmaceutically acceptable salts are well known in the art. For example, S. M. Berge, et al. (J. Pharmaceutical Sciences, 66: 1-19 (1977)), incorporated herein by reference in its entirety, describes various pharmaceutically acceptable salts in details.

The term "infection" or "bacterial infection" as used herein refers to and includes presence of bacteria, in or on a subject, which, if its growth were inhibited, would result in a benefit to the subject. As such, the term "infection" in addition to referring to the presence of bacteria also refers to normal flora, which is not desirable. The term "infection" includes infection caused by bacteria.

The term "treat", "treating" or "treatment" as used herein refers to administering a medicament, including a pharmaceutical composition, or one or more pharmaceutically active ingredients, for prophylactic and/or therapeutic purposes. The term "prophylactic treatment" refers to treating a subject who is not yet infected, but who is susceptible to, or otherwise at a risk of infection (preventing the bacterial infection). The term "therapeutic treatment" refers to administering treatment to a subject already suffering from infection. The terms "treat", "treating" or "treatment" as used herein also refer to administering compositions, or one or more of pharmaceutically active ingredients discussed herein, with or without additional pharmaceutically active or inert ingredients, in order to: (i) reduce or eliminate either a bacterial infection, or one or more symptoms of the bacterial infection, or (ii) retard the progression of a bacterial infection, or of one or more symptoms of the bacterial infection, or (iii) reduce the severity of a bacterial infection, or of one or more symptoms of the bacterial infection, or (iv) suppress the clinical manifestation of a bacterial infection, or (v) suppress the manifestation of adverse symptoms of the bacterial infection.

The term "pharmaceutically effective amount" or "therapeutically effective amount" or "effective amount" as used herein refers to an amount, which has a therapeutic effect or is the amount required to produce a therapeutic effect in a subject. For example, a therapeutically or pharmaceutically effective amount of an antibacterial agent or a pharmaceutical composition is the amount of the antibacterial agent or the pharmaceutical composition required to produce a desired therapeutic effect as may be judged by clinical trial results, model animal infection studies, and/or in vitro studies (e.g. in agar or broth media). The pharmaceutically effective amount depends on several factors, including but not limited to, the microorganism (e.g. bacteria) involved, characteristics of the subject (for example height, weight, sex, age and medical history), severity of infection and the particular type of the antibacterial agent used. For prophylactic treatments, a therapeutically or prophylactically effective amount is that amount which would be effective in preventing a microbial (e.g. bacterial) infection. The compounds and/or pharmaceutical compositions according to the invention are used in amounts that are effective in providing the desired therapeutic effect or result.

The term "administration" or "administering" includes delivery of a composition, or one or more pharmaceutically active ingredients to a subject, including for example, by any appropriate methods, which serves to deliver the composition or its active ingredients or other pharmaceutically active ingredients to the site of the infection. The method of administration may vary depending on various factors, such as for example, the components of the pharmaceutical composition or the nature of the pharmaceutically active or inert ingredients, the site of the potential or actual infection, the microorganism involved, severity of the infection, age and physical condition of the subject and a like. Some non-limiting examples of ways to administer a composition or a pharmaceutically active ingredient to a subject according to this invention includes oral, intravenous, topical, intra-respiratory, intra-peritoneal, intra-muscular, parenteral, sublingual, transdermal, intranasal, aerosol, intra-ocular, intra-tracheal, intra-rectal, vaginal, gene gun, dermal patch, eye drop, ear drop or mouthwash. In case of a pharmaceutical composition comprising more than one ingredient (active or inert), one of way of administering such composition is by admixing the ingredients (e.g. in the form of a suitable unit dosage form such as tablet, capsule, solution, powder and a like) and then administering the dosage form. Alternatively, the ingredients may also be administered separately (simultaneously or one after the other) as long as these ingredients reach beneficial therapeutic levels such that the composition as a whole provides a synergistic and/or the desired effect.

The term "growth" as used herein refers to a growth of one or more microorganisms and includes reproduction or population expansion of the microorganism (e.g. bacteria). The term "growth" also includes maintenance of on-going metabolic processes of a microorganism (e.g. bacteria), including processes that keep the microorganism alive.

The term, "effectiveness" as used herein refers to ability of a treatment or a composition or one or more pharmaceutically active ingredients to produce a desired biological effect in a subject. For example, the term "antibacterial effectiveness" of a composition or an antibacterial agent refers to the ability of the composition or the antibacterial agent to treat or prevent the microbial (e.g. bacterial) infection in a subject.

The term "synergistic" or "synergy" as used herein refers to the interaction of two or more agents so that their combined effect is greater than their individual effects.

The term "pharmaceutically inert ingredient", "inactive ingredient", "carrier" or "excipient" refers to a compound or material used to facilitate administration of a compound, including for example, to increase the solubility of the compound. Typical, non-limiting examples of solid carriers include, starch, lactose, dicalcium phosphate, sucrose, and kaolin and so on. Typical, non-limiting examples of liquid carriers include sterile water, saline, buffers, non-ionic surfactants, and edible oils such as oil, peanut and sesame oils and so on. In addition, various adjuvants commonly used in the art may be included. These and other such compounds are described in the literature, for example, in the Merck Index (Merck & Company, Rahway, N.J.). Considerations for inclusion of various components in pharmaceutical compositions are described, for example, in Gilman et al. (Eds.) (1990); Goodman and Gilman's: The Pharmacological Basis of Therapeutics, 8th Ed., Pergamon Press., which is incorporated herein by reference in its entirety.

The term "subject" as used herein refers to a vertebrate or invertebrate, including a mammal. The term "subject" includes human, animal, a bird, a fish, or an amphibian. Typical, non-limiting examples of a "subject" includes humans, cats, dogs, horses, sheep, bovine cows, pigs, lambs, rats, mice and guinea pigs.

The term "parenteral administration" refers to and includes a route of administration that does not involve gastrointestinal tract directly. Typical, non-limiting examples of parenteral route of administration includes intravenous (into a vein), intra-arterial (into an artery), intraosseous infusion (into the bone marrow), intra-muscular, intracerebral, intrathecal, subcutaneous administration. In general, the parenteral administration is performed by injecting or infusing the composition or the active ingredient(s) directly into a subject without direct involvement of the gastrointestinal tract.

In one general aspect, there is provided a pharmaceutical composition in the form of a solution, said solution comprising S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt, said solution having pH within the range of about 8 to about 11. In some embodiments, the solution has pH within the range of about 9 to about 10.5.

The compositions according to the invention are in the form of a solution and can be obtained by, for example, dissolving S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt, in a pharmaceutically acceptable liquid diluent. Alternatively, the compositions according to the invention can also be obtained by dissolving any other compound capable of providing S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt when dissolved in a pharmaceutically acceptable diluent. Typical, non-limiting example of such other compounds include a various hydrate forms of S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt, including the tetrahydrate form. Typical, non-limiting examples of such liquid diluent include water, sodium chloride solution, dextrose solution, saline solution and a like. In some embodiment, the composition according to the invention is obtained by dissolving S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt in water. In some other embodiments, the composition according to the invention is obtained by dissolving S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt tetrahydrate from in water.

In solution form, S-(-)-9-fluoro-6,7-dihydro-8-(4-hydroxypiperidin-1-yl)-5-methyl-1-oxo-1H,5H-benzo[i,j] quinolizine-2-carboxylic acid L-arginine may be present as such or in a dissociated form. Both these and any other forms are intended to be covered within the scope of the invention.

The compositions according to the invention are in the form of a solution adapted to maintain pH within the range of 9 to 10.5. In general, the pH is maintained at a desired level by addition of suitable pH adjusting agent to the composition. In some embodiments, the pH is maintained at a desired level by addition of a buffer. In some embodiments, the pH is maintained at a desired level by addition of a sufficient quantity of L-arginine or a pharmaceutically acceptable salt thereof to the composition.

The amount of active and inactive ingredients in the composition according to the invention can vary depending on specific requirements. However, all active and inactive ingredients in the composition according to the invention are present in therapeutically effective amounts, such that the composition as a whole is therapeutically effective.

In some embodiments, not covered by the claimed invention, S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt is present in the composition within the range of about 200 mg to about 2000 mg.

In some other embodiments, not covered by the claimed invention, S-(-)-9-fluoro-6,7-dihydro-8-(4-hydroxypiperidin-1-yl)-5-methyl-1-oxo-1H,5H-benzo[i,j] quinolizine -2-carboxylic acid L-arginine salt is present in the composition within the range of about 400 mg to about 1500 mg.

In some embodiments, there are provided pharmaceutical compositions in the form of a solution, said solution having pH within the range of about 9 to about 10.5; said solution comprising:
(a) S-(-)-9-fluoro-6,7-dihydro-8-(4-hydroxypiperidin-1 -yl)-5-methyl-1-oxo-1H,5H-benzo[i,j] quinolizine -2-carboxylic acid L-arginine salt, (b) L-arginine or a pharmaceutically acceptable salt thereof, and (c) sodium chloride.

In some embodiments, the pharmaceutical compositions according to the invention comprise L-arginine or a pharmaceutically acceptable salt thereof. In some embodiments, not covered by the claimed invention, L-arginine or a pharmaceutically acceptable salt thereof is present in the composition within the range of about 200 mg to about 2000 mg. In some other embodiments, not covered by the claimed invention, L-arginine or a pharmaceutically acceptable salt is present in the composition within the range of about 400 mg to about 1500 mg.

In some embodiments, the pharmaceutical compositions according to the invention comprise sodium chloride. In some other embodiments not covered by the claimed invention, , sodium chloride is present in the composition within the range of about 200 mg to about 2000 mg. In some other embodiments, not covered by the claimed invention sodium chloride is present in the composition within the range of about 400 mg to about 1500 mg.

In some embodiments, not covered by the claimed invention there are provided pharmaceutical compositions in the form of a 100 ml solution, said solution having pH within the range of about 8 to about 11; said solution comprising:
(a) about 200 to about 2000 mg of S-(-)-9-fluoro-6,7-dihydro-8-(4-hydroxypiperidin-1-yl)-5-methyl-1-oxo-1H,5H-benzo[i,j] quinolizine -2-carboxylic acid L-arginine salt, (b) about 200 to about 2000 mg of L-arginine or a pharmaceutically acceptable salt thereof, and (c) about 200 to about 2000 mg of sodium chloride.

In some embodiments, not covered by the claimed invention there are provided pharmaceutical compositions in the form of a 100 ml solution, said solution having pH within the range of about 9 to about 10.5; said solution comprising: (a) about 200 to about 2000 mg of S-(-)-9-fluoro-6,7-dihydro-8-(4-hydroxypiperidin-1-yl)-5-methyl-1-oxo-1H,5H-benzo[i,j] quinolizine -2-carboxylic acid L-arginine salt, (b) about 200 to about 2000 mg of L-arginine or a pharmaceutically acceptable salt thereof, and (c) about 200 to about 2000 mg of sodium chloride.

In some embodiments, not covered by the claimed invention the compositions according to the invention comprise about 2 mg/ml to about 20 mg/ml of S-(-)-9-fluoro-6,7-dihydro-8-(4-hydroxypiperidin-1-yl)-5-methyl-1-oxo-1H,5H-benzo[i,j] quinolizine -2-carboxylic acid L-arginine salt. In some other embodiments, not covered by the claimed invention the compositions according to the invention comprise about 2 mg/ml to about 20 mg/ml of L-arginine or a pharmaceutically acceptable salt thereof. In some other embodiments, not covered by the claimed invention the compositions according to the invention comprise about 2 mg/ml to about 20 mg/ml of sodium chloride.

In some embodiments, the compositions according to the invention have osmolarity within the range of about 250 to about 350 mOsm/L.

The osmolarity may be adjusted to a desired level by addition of a suitable substance capable of modifying the osmolarity. In some embodiments, the osmolarity is maintained in the desired range by addition of sodium chloride.

The pharmaceutical compositions according to the invention may include one or more pharmaceutically acceptable carriers or excipients or a like. Typical, non-limiting examples of such carriers or excipients include mannitol, lactose, glucose, sucrose, emulsifying agents, solubilizing agents, pH buffering agents, stabilizing agents and a like.

The compositions according to the invention are stable over a wide range of storage conditions. In some embodiments, the compositions according to the invention comprise less than 5% w/w of total impurities. In some embodiments, the compositions according to the invention comprise less than 5% w/w of total impurities after storage at a temperature of about 40 ± 2°C and a relative humidity of about 75 ± 5 % for 3 months. In some embodiments, the compositions according to the invention comprise less than 5% w/w of total impurities after storage at a temperature of about 25 ± 2°C and a relative humidity of about 60 ± 5 % for 24 months.

In some embodiments, the compositions according to the invention comprise one or more of the following:
(i) less than 0.25% w/w of 9-fluoro-8-hydroxy-5-methyl-6,7-dihydro-1-oxo-1H,5H-benzo [i,j]quinolizine-2-carboxylic acid;
(ii) less than 3% w/w of 9-fluoro-6,7-dihydro-8-amino-5-methyl-1-oxo -1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H, 5H-benzo[i,j]quinolizine-2-carboxylic acid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

In some other embodiments, the compositions according to the invention comprise one or more of the following after storage at a temperature of about 40 ± 2°C and a relative humidity of about 75 ± 5 % for 3 months:
(i) less than 0.25% w/w of 9-fluoro-8-hydroxy-5-methyl-6,7-dihydro-1-oxo-1H,5H-benzo [i,j]quinolizine-2-carboxylic acid;
(ii) less than 3% w/w of 9-fluoro-6,7-dihydro-8-amino-5-methyl-1-oxo-1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H, 5H-benzo[i,j]quinolizine-2-carboxylic acid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

In some other embodiments, the compositions according to the invention comprise one or more of the following after storage at a temperature of about 25 ± 2°C and a relative humidity of about 60 ± 5 % for 24 months:
(i) less than 0.25% w/w of 9-fluoro-8-hydroxy-5-methyl-6,7-dihydro-1-oxo-1H,5H-benzo [i,j]quinolizine -2-carboxylic acid;
(ii) less than 3% w/w of 9-fluoro-6,7-dihydro-8-amino-5-methyl-1-oxo -1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H, 5H-benzo[i,j]quinolizine-2-carboxylic acid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

In some embodiments, the pharmaceutical compositions according to the invention are present in the form of a solution that can be reconstituted by addition of a compatible reconstitution diluent prior to administration. In some embodiments, the pharmaceutical compositions according to the invention are present in the form of ready-to-use solution for parenteral administration. In some embodiments, the pharmaceutical compositions according to the invention are present in the form of a solution as a unit dose contained in a bottle, vial or bag prior to parenteral administration. In some other embodiments, the pharmaceutical compositions according to the invention are in the form of a frozen composition that can be diluted with a compatible reconstitution diluent prior to administration. A wide variety of reconstitution diluents can be used. Typical, non-limiting example of reconstitution diluent includes water for injection, 0.9% sodium chloride solution, 5% dextrose solution, normal saline solution and a like.

In another general aspect, the compositions according to the invention are useful in treatment, control or prevention of a bacterial infection. In some embodiments, the compositions according to the invention are used in the preparation of a medicament for treatment, control, or prevention of bacterial infection.

In another general aspect, there is provided a method for treatment, control and/or prevention of bacterial infection in a subject, said method comprising administering to said subject a composition according to the invention. In some embodiments, the composition is administered parenterally.

### EXAMPLES

The following examples illustrate the embodiments of the invention that are presently best known. However, it is to be understood that the following are only exemplary or illustrative of the application of the principles of the present invention.

### Example 1

Various ingredients used in the following example are summarized below:
**Compound A:**
   S-(-)-9-fluoro-6,7-dihydro-8-(4-hydroxypiperidin-1-yl)-5-methyl-1-oxo-1H,5H-benzo[i,j] quinolizine-2-carboxylic acid L-arginine salt.
**Compound B:**
   9-fluoro-8-hydroxy-5-methyl-6,7-dihydro-1-oxo-1H,5H-benzo[i,j]quinolizine-2-carboxylic acid.
**Compound C:**
   9-fluoro-6,7-dihydro-8-amino-5-methyl-1-oxo-1H,5H-benzo[i,j] quinolizine-2-carboxylic acid.
**Compound D:**
   8-fluoro-9-(4-hydroxy-piperidin-1-yl)-5-methyl-6,7-dihydro-1-oxo-1H,5H-benzo[i,j]quinolizine-2-carboxylic acid.
**Compound E:**
   8-(4-hydroxy-1-piperidinyl)-5-methyl-6,7-dihydro-1-oxo-1H,5H-benzo[i,j]quinolizine-2-carboxylic acid.
**Compound F:**
   8,9-difluoro-5-methyl-6,7-dihydro-1-oxo-1H,5H-benzo[i,j]quinolizine-2-carboxylic acid.
**Compound G:**
   R-(+)-9-fluoro-8-(4-hydroxy-piperidin-1-yl)-5-methyl-6,7-dihydro-1-oxo-1H,5H-benzo[i,j] quinolizine-2-carboxylic acid.

A wide variety of compositions comprising various ingredients (active as well as inactive ingredients) were prepared. The amount of such ingredients (active as well as inactive) in these compositions was within the ranges as described. The pH and osmolarity of these compositions varied over a wide range, including those described herein. Details of a few examples are given in Table 1, below, whereas composition 2 does not fall within the invention claimed.

| **Table 1** | | |
|---|---|---|
| **Ingredients** | **Composition 1** | **Composition 2** |
| Compound A | 1186.7 mg | 890 mg |
| L-Arginine | 1333 mg | 1000 mg |
| Sodium Chloride | 580 mg | 670 mg |
| Nitrogen | q.s. to purge | q.s. to purge |
| Water for Injection | q.s. to 100 ml | q.s. to 100 ml |

In general, L-Arginine, Compound A, and sodium chloride were sequentially added to appropriate amount of water (water for injection having dissolved oxygen content less than 2 ppm). The bulk solution was filtered using 1.2µ PP filter and 02µ PES filters. The filtered solution was packed into bottle and sterilized. The stability of the prepared compositions was determined at appropriate conditions and the results are given in Table 2 (for Composition 1) and Table 3 (for Composition 2).

| **Table 2** | | | | |
|---|---|---|---|---|
| **Sr.** | **Test** | **Values** | | |
| | | Initial | After storage at 40 ± 2°C and 75 ± 5% relative humidity for 3 months | After storage at 25 ± 2°C and 60 ± 5% relative humidity for 24 months |
| 1. | Assay of Compound A | 98.7 | 100.4 | 100.1 |
| 2. | Assay of NaCl | 100.7 | 100.7 | 99.3 |
| 3. | ***Related substances** as **determined by HPLC*** | | | |
| 4. | Compound B | 0.000 | 0.000 | 0.000 |
| 5. | Compound C | 0.138 | 0.381 | 0.809 |
| 6. | Compound D | 0.000 | 0.000 | 0.000 |
| 7. | Compound E | 0.019 | 0.053 | 0.023 |
| 8. | Compound F | 0.000 | 0.000 | 0.000 |
| 9. | Compound G | 0.000 | 0.000 | 0.000 |
| 10. | Total impurities | 0.157 | 0.503 | 0.935 |
| 11. | pH of the composition | 9.67 | 9.50 | 9.73 |
| 12. | Osmolarity (mOsm/L) | 291 | 291 | 296 |

| **Table 3** | | | | |
|---|---|---|---|---|
| **Sr.** | **Test** | **Values** | | |
| | | Initial | After storage at 40 ± 2°C and 75 ± 5% relative humidity for 3 months | After storage at 25 ± 2°C and 60 ± 5% relative humidity for 24 months |
| 1. | pH of the composition | 9.42 | 9.65 | 9.58 |
| 2. | Assay of Compound A | 102.8 | 101.5 | 102.7 |
| 3. | Assay of NaCl | 100.98 | 102.1 | 100.0 |
| 4. | ***Related substances (by HPL C)*** | | | |
| 5. | Compound B | 0.000 | 0.000 | 0.005 |
| 6. | Compound C | 0.131 | 0.508 | 0.608 |
| 7. | Compound D | 0.000 | 0.000 | 0.000 |
| 8. | Compound E | 0.049 | 0.049 | 0.057 |
| 9. | Compound F | 0.000 | 0.000 | 0.000 |
| 10. | Compound G | 0.000 | 0.000 | 0.000 |
| 11. | Total impurities | 0.180 | 0.624 | 0.777 |

## Claims

1. A pharmaceutical composition in the form of an aqueous 100 ml solution, said solution having pH within the range of 9 to 10.5 and said solution has osmolarity within the range of about 250 to about 350 mOsm/L; said solution comprising: (a) 1186.7 mg of S-(-)-9-fluoro-6,7-dihydro-8-(4-hydroxypiperidin-1-yl)-5-methyl-1-oxo-1H,5H-benzo [i,j] quinolizine -2-carboxylic acid L-arginine salt, (b) 1333 mg of L-arginine or a pharmaceutically acceptable salt thereof, and (c) 580 mg of sodium chloride.

2. The composition according to Claim 1, said composition comprising less than 5% w/w of total impurities.

3. The composition according to Claim 1, said composition comprising less than 5% w/w of total impurities after storage at a temperature of about 40 ± 2°C and a relative humidity of about 75 ± 5 % for 3 months.

4. The composition according to Claim 1, said composition comprising less than 5% w/w of total impurities after storage at a temperature of about 25 ± 2°C and a relative humidity of about 60 ± 5 % for 24 months.

5. The composition according to Claim 1, said composition comprising one or more of the following:
(i) less than 0.25% w/w of 9-fluoro-8-hydroxy-5-methyl-6,7-dihydro-1-oxo-1H,5H-benzo[i,j]quinolizine -2-carboxylic acid;
(ii) less than 3% w/w of 9-fluoro-6,7-dihydro-8-amino-5-methyl-1-oxo -1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H,5H-benzo[i,j]quinolizine-2-carboxylicacid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

6. The composition according to Claim 1, said composition comprising one or more of the following after storage at a temperature of about 40 ± 2°C and a relative humidity of about 75 ± 5 % for 3 months:
(i) less than 0.25% w/w of 9-fluoro-8-hydroxy-5-methyl-6,7-dihydro-1-oxo-1H,5H-benzo[i,j]quinolizine-2-carboxylic acid;
(ii) less than 3% w/w of 9-fluoro-6,7-dihydro-8-amino-5-methyl-1-oxo -1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H,5H-benzo[i,j]quinolizine-2-carboxylicacid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

7. The composition according to Claim 1, said composition comprising one or more of the following after storage at a temperature of about 25 ± 2°C and a relative humidity of about 60 ± 5 % for 24 months:
(i) less than 0.25% w/w of 9-fluoro-8-hydroxy-5-methyl-6,7-dihydro-1-oxo-1H,5H-benzo[i,j]quinolizine -2-carboxylic acid;
(ii) less than 3% w/w of 9-fluoro-6,7-dihydro-8-amino-5-methyl-1-oxo -1H,5H-benzo[i,j] quinolizine-2-carboxylic acid;
(iii) less than 0.25% w/w of 8-fluoro-9-(4-hydroxy-piperidin-1-yl)-5-methyl-6,7-dihydro-1-oxo-1H,5H-benzo[i,j]quinolizine-2-carboxylic acid;
(iv) less than 0.25% w/w of 8-(4-hydroxy-1-piperidinyl)-5-methyl-6,7-dihydro-1-oxo-1H,5H-benzo[i,j]quinolizine-2-carboxylicacid;
(v) less than 0.25% w/w of 8,9-difluoro-5-methyl-6,7-dihydro-1-oxo-1H,5H-benzo[i,j] quinolizine-2-carboxylic acid; or
(vi) less than 0.5% w/w of R-(+)-9-fluoro-8-(4-hydroxy-piperidin-1-yl)-5-methyl-6,7-dihydro-1-oxo-1H,5H-benzo[i,j]quinolizine-2-carboxylic acid.

8. Composition according to any of the claims 1-7 for us in a method for treating, controlling or preventing bacterial infection.

9. Composition for use according to Claim 8, wherein the composition is administered parenterally.

## Patentansprüche

1. Pharmazeutische Zusammensetzung in Form einer wässrigen 100-ml-Lösung, wobei besagte Lösung einen pH-Wert innerhalb des Bereichs von 9 bis 10,5 hat und besagte Lösung eine Osmolarität innerhalb des Bereichs von ca. 250 bis ca. 350 mOsm/L hat; wobei besagte Lösung Folgendes umfasst: (a) 1186,7 mg S-(-)-9-Fluor-6,7-dihydro-8-(4-hydroxypiperidin-1-yl)-5-methyl-1-oxo-1H,5H-benzo[i,j]chinolizin-2-carbonsäure-L-Arginin-Salz, (b) 1333 mg L-Arginin oder ein pharmazeutisch verträgliches Salz davon und (c) 580 mg Natriumchlorid.

2. Zusammensetzung nach Anspruch 1, wobei besagte Zusammensetzung weniger als 5 Gew.-% der gesamten Verunreinigungen umfasst.

3. Zusammensetzung nach Anspruch 1, wobei besagte Zusammensetzung weniger als 5 Gew.-% der gesamten Verunreinigungen nach dem Lagern bei einer Temperatur von ca. 40 ± 2°C und einer relativen Luftfeuchtigkeit von ca. 75 ± 5 % für 3 Monate umfasst.

4. Zusammensetzung nach Anspruch 1, wobei besagte Zusammensetzung weniger als 5 Gew.-% der gesamten Verunreinigungen nach dem Lagern bei einer Temperatur von ca. 25 ± 2°C und einer relativen Luftfeuchtigkeit von ca. 60 ± 5 % für 24 Monate umfasst.

5. Zusammensetzung nach Anspruch 1, wobei besagte Zusammensetzung eines oder mehreres von Folgendem umfasst:
(i) weniger als 0,25 Gew.-% der 9-Fluor-8-hydroxy-5-methyl-6,7-dihydro-1-oxo-1H,5H-benzo [i,j]chinolizin-2-carbonsäure;
(ii) weniger als 3 Gew.-% der 9-Fluor-6,7-dihydro-8-amino-5-methyl-1-oxo-1H,5H-benzo[i,j] chinolizin-2-carbonsäure;
(iii) weniger als 0,25 Gew.-% der 8-Fluor-9-(4-hydroxy-piperidin-1-yl)-5-methyl-6,7-dihydro-1-oxo-1H,5H-benzo[i,j]chinolizin-2-carbonsäure;
(iv) weniger als 0,25 Gew.-% der 8-(4-Hydroxy-1-piperidinyl)-5-methyl-6,7-dihydro-1-oxo-1H,5H-benzo[i,j]chinolizin-2-carbonsäure;
(v) weniger als 0,25 Gew.-% der 8,9-Difluor-5-methyl-6,7-dihydro-1-oxo-1H,5H-benzo[i,j] chinolizin-2-carbonsäure; oder
(vi) weniger als 0,5 Gew.-% der R-(+)-9-Fluor-8-(4-hydroxy-piperidin-1-yl)-5-methyl-6,7-dihydro-1-oxo-1H,5H-benzo[i,j]chinolizin-2-carbonsäure.

6. Zusammensetzung nach Anspruch 1, wobei besagte Zusammensetzung nach dem Lagern bei einer Temperatur von ca. 40 ± 2°C und einer relativen Luftfeuchtigkeit von ca. 75 ± 5 % für 3 Monate eines oder mehreres von Folgendem umfasst:
(i) weniger als 0,25 Gew.-% der 9-Fluor-8-hydroxy-5-methyl-6,7-dihydro-1-oxo-1H,5H-benzo [i,j]chinolizin-2-carbonsäure;
(ii) weniger als 3 Gew.-% der 9-Fluor-6,7-dihydro-8-amino-5-methyl-1-oxo-1H,5H-benzo[i,j] chinolizin-2-carbonsäure;
(iii) weniger als 0,25 Gew.-% der 8-Fluor-9-(4-hydroxy-piperidin-1-yl)-5-methyl-6,7-dihydro-1-oxo-1H,5H-benzo[i,j]chinolizin-2-carbonsäure;
(iv) weniger als 0,25 Gew.-% der 8-(4-Hydroxy-1-piperidinyl)-5-methyl-6,7-dihydro-1-oxo-1H,5H-benzo[i,j]chinolizin-2-carbonsäure;
(v) weniger als 0,25 Gew.-% der 8,9-Difluor-5-methyl-6,7-dihydro-1-oxo-1H,5H-benzo[i,j] chinolizin-2-carbonsäure; oder
(vi) weniger als 0,5 Gew.-% der R-(+)-9-Fluor-8-(4-hydroxy-piperidin-1-yl)-5-methyl-6,7-dihydro-1-oxo-1H,5H-benzo[i,j]chinolizin-2-carbonsäure.

7. Zusammensetzung nach Anspruch 1, wobei besagte Zusammensetzung nach dem Lagern bei einer Temperatur von ca. 25 ± 2°C und einer relativen Luftfeuchtigkeit von ca. 60 ± 5 % für 24 Monate eines oder mehreres von Folgendem umfasst:
(i) weniger als 0,25 Gew.-% der 9-Fluor-8-hydroxy-5-methyl-6,7-dihydro-1-oxo-1H,5H-benzo [i,j]chinolizin-2-carbonsäure;
(ii) weniger als 3 Gew.-% der 9-Fluor-6,7-dihydro-8-amino-5-methyl-1-oxo -1H,5H-benzo[i,j] chinolizin-2-carbonsäure;
(iii) weniger als 0,25 Gew.-% der 8-Fluor-9-(4-hydroxy-piperidin-1-yl)-5-methyl-6,7-dihydro-1-oxo-1H,5H-benzo[i,j]chinolizin-2-carbonsäure;
(iv) weniger als 0,25 Gew.-% der 8-(4-Hydroxy-1-piperidinyl)-5-methyl-6,7-dihydro-1-oxo-1H,5H-benzo[i,j]chinolizin-2-carbonsäure;
(v) weniger als 0,25 Gew.-% der 8,9-Difluor-5-methyl-6,7-dihydro-1-oxo-1H,5H-benzo[i,j] chinolizin-2-carbonsäure; oder
(vi) weniger als 0,5 Gew.-% der R-(+)-9-Fluor-8-(4-hydroxy-piperidin-1-yl)-5-methyl-6,7-dihydro-1-oxo-1H,5H-benzo[i,j]chinolizin-2-carbonsäure.

8. Zusammensetzung nach einem der Ansprüche 1-7 zum Verwenden in einem Verfahren zum Behandeln, Steuern oder Verhindern einer bakteriellen Infektion.

9. Zusammensetzung zum Verwenden nach Anspruch 8, wobei die Zusammensetzung parenteral verabreicht wird.

## Revendications

1. Composition pharmaceutique sous la forme d'une solution aqueuse de 100 ml, ladite solution ayant un pH compris dans la gamme de 9 à 10,5 et ladite solution ayant une osmolarité dans la gamme d'environ 250 à environ 350 mOsm/L ; ladite solution comprenant : (a) 1186,7 mg de sel de L-arginine de l'acide S-(-)-9-fluoro-6,7-dihydro-8-(4-hydroxypipéridin-1-yl)-5-méthyl-1-oxo-1H,5H-benzo[i,j]quinolizine-2-carboxylique, (b) 1333 mg de L-arginine ou d'un sel pharmaceutiquement acceptable de celle-ci, et (c) 580 mg de chlorure de sodium.

2. Composition selon la revendication 1, ladite composition comprenant moins de 5% p/p d'impuretés totales.

3. Composition selon la revendication 1, ladite composition comprenant moins de 5% p/p d'impuretés totales après stockage à une température d'environ 40 ± 2°C et une humidité relative d'environ 75 ± 5 % pendant 3 mois.

4. Composition selon la revendication 1, ladite composition comprenant moins de 5% p/p d'impuretés totales après stockage à une température d'environ 25 ± 2°C et une humidité relative d'environ 60 ± 5 % pendant 24 mois.

5. Composition selon la revendication 1, ladite composition comprenant un ou plusieurs des éléments suivants :
(i) moins de 0,25% p/p d'acide 9-fluoro-8-hydroxy-5-méthyl-6,7-dihydro-1-oxo1-H,5H-benzo[i,j]quinolizine-2-carboxylique;
(ii) moins de 3% p/p d'acide 9-fluoro-6,7-dihydro-8-amino-5-méthyl-1-oxo-1H,5H-benzo[i,j]quinolizine-2-carboxylique ;
(iii) moins de 0,25% p/p d'acide 8-fluoro-9-(4-hydroxy-pipéridin-1-yl)-5-méthyl-6,7dihydro-1-oxo-1 H,5H-benzo[i,j]quinolizine-2-carboxylique ;
(iv) moins de 0,25% p/p d'acide 8-(4-hydroxy-1-pipéridinyl)-5-méthyl-6,7-dihydro-1-oxo-1H,5H-benzo[i,j]quinolizine-2-carboxylique ;
(v) moins de 0,25% p/p d'acide 8,9-difluoro-5-méthyl-6,7-dihydro-1-oxo-1H,5H-benzo[i,j]quinolizine-2-carboxylique ; ou
(vi) moins de 0,5% p/p d'acide R-(+)-9-fluoro-8-(4-hydroxy-pipéridin-1-yl)-5-méthyl-6,7-dihydro-1-oxo-1H,5H-benzo[i,j] quinolizine-2-carboxylique.

6. Composition selon la revendication 1, ladite composition comprenant un ou plusieurs des éléments suivants après stockage à une température d'environ 40 ± 2°C et une humidité relative d'environ 75 ± 5 % pendant 3 mois :
(i) moins de 0,25% p/p d'acide 9-fluoro-8-hydroxy-5-méthyl-6,7-dihydro-1-oxo-1 H,5H-benzo[i,j]quinolizine-2-carboxylique ;
(ii) moins de 3% p/p d'acide 9-fluoro-6,7-dihydro-8-amino-5-méthyl-1-oxo-1H,5H-benzo[i,j]quinolizine-2-carboxylique ;
(iii) moins de 0,25% p/p d'acide 8-fluoro-9-(4-hydroxy-pipéridin-1-yl)-5-méthyl-6,7-dihydro-1-oxo-1 H,5H-benzo[i,j]quinolizine-2-carboxylique ;
(iv) moins de 0,25% p/p d'acide 8-(4-hydroxy-1-pipéridinyl)-5-méthyl-6,7-dihydro-1-oxo-1H,5H-benzo[i,j]quinolizine-2-carboxylique ;
(v) moins de 0,25% p/p d'acide 8,9-difluoro-5-méthyl-6,7-dihydro-1-oxo-1H,5H-benzo[i,j]quinolizine-2-carboxylique ; ou
(vi) moins de 0,5% p/p d'acide R-(+)-9-fluoro-8-(4-hydroxy-pipéridin-1-yl)-5-méthyl-6,7-dihydro-1-oxo-1H,5H-benzo[i,j]quinolizine-2-carboxylique.

7. Composition selon la revendication 1, ladite composition comprenant un ou plusieurs des éléments suivants après stockage à une température d'environ 25 ± 2°C et une humidité relative d'environ 60 ± 5 % pendant 24 mois :
(i) moins de 0,25% p/p d'acide 9-fluoro-8-hydroxy-5-méthyl-6,7-dihydro-1-oxo-1 H,5H-benzo[i,j]quinolizine-2-carboxylique ;
(ii) moins de 3% p/p d'acide 9-fluoro-6,7-dihydro-8-amino-5-méthyl-1-oxo-1H,5H-benzo[i,j]quinolizine-2-carboxylique ;
(iii) moins de 0,25% p/p d'acide 8-fluoro-9-(4-hydroxy-pipéridin-1-yl)-5-méthyl-6,7-dihydro-1-oxo-1 H,5H-benzo[i,j]quinolizine-2-carboxylique ;
(iv) moins de 0,25% p/p d'acide 8-(4-hydroxy-1-pipéridinyl)-5-méthyl-6,7-dihydro-1-oxo-1H,5H-benzo[i,j]quinolizine-2-carboxylique ;
(v) moins de 0,25% p/p d'acide 8,9-difluoro-5-méthyl-6,7-dihydro-1-oxo-1H,5H-benzo[i,j]quinolizine-2-carboxylique ; ou
(vi) moins de 0,5% p/p d'acide R-(+)-9-fluoro-8-(4-hydroxy-pipéridin-1-yl)-5-méthyl-6,7-dihydro-1-oxo-1H,5H-benzol[i,j]quinolizine-2-carboxylique.

8. Composition selon l'une quelconque des revendications 1 à 7 destinée à être utilisée dans un procédé de traitement, de contrôle ou de prévention d'une infection bactérienne.

9. Composition destinée à être utilisée selon la revendication 8, où la composition est administrée par voie parentérale.
